# EUROPEAN PATENT SPECIFICATION

(11) **EP 4 051 099 B1**
(45) Date of publication and mention of the grant of the patent: **08.04.2026**
(21) Application number: 20881584.5
(22) Date of filing: 28.10.2020
(51) Int. Cl.: A61B 5/02, A41D 13/12, A61B 5/00, A61B 5/0205, A61B 5/024, A61B 5/08, A61B 5/256, A61B 5/27, A61B 5/282, A61B 5/11

(54) **PADDED, FLEXIBLE ENCASING FOR BODY MONITORING SYSTEMS IN FABRICS**
GEPOLSTERTE, FLEXIBLE UMHÜLLUNG FÜR KÖRPERÜBERWACHUNGSSYSTEME IN GEWEBEN
ENVELOPPE SOUPLE REMBOURRÉE POUR SYSTÈMES DE SURVEILLANCE CORPORELLE DANS DES TISSUS

(30) Priority: 28.10.2019 US 201916665669
(43) Date of publication of application: 07.09.2022
(73) Proprietor: Bloomer Health Tech, Inc., Boston, MA 02116 (US)
(72) Inventor: CHONG RODRIGUEZ, Alicia, Dedham, MA 02026 (US); ABRACA ABRACA, Monica, Lima, 43 (PE); HALABY, Aceil, Wayyland, MA 01778 (US)
(74) Representative: Müller-Boré & Partner Patentanwälte PartG mbB
(86) International application number: PCT/US2020/057735
(87) International publication number: WO 2021/086963

(56) References cited:
- WO-A1-2018/148756
- WO-A1-2019/158933
- CA-A1- 3 002 253
- US-A1- 2010 130 846
- US-A1- 2016 296 135
- US-A1- 2017 049 164
- US-A1- 2018 078 155
- US-A1- 2018 317 845
- US-A1- 2018 317 845
- US-A1- 2019 206 538

## Description

### CROSS-REFERENCE TO RELATED APPLICATIONS

This application claims the benefit of priority of U.S. Non-Provisional Application Serial No. 16/665,669, filed on October 28, 2019, and entitled "PADDED, FLEXIBLE ENCASING FOR BODY MONITORING SYSTEMS IN FABRICS." FIELD OF THE INVENTION

The present invention generally relates to the field of wearable devices. In particular, the present invention is directed to a padded, flexible encasing for body monitoring systems in fabrics.

### BACKGROUND

Cardiovascular diseases cause more deaths globally than cancer, HIV and malaria combined. Infections, metabolic, respiratory and cardiac diseases, cancers and other illnesses are all massive problems that can be better tackled with continuous and event monitoring and personalized information of the patient for an efficient diagnosis.

US 2018317845 A1 discloses the preamble of claim 1.

### SUMMARY OF THE DISCLOSURE

The present invention is set out in the appended set of claims.

According to a first aspect, the present invention provides a garment having a wearable monitoring device, the garment comprising: the wearable monitoring device coupled to the garment, wherein: the wearable monitoring device comprises a circuit board having a first side and a second side opposite the first side; the circuit board comprises an integrated circuit configured to process physiological measurement and wirelessly transmit the physiological measurements and biometric measurements to another computing device; at least one soft-based module coupled to the wearable monitoring device and the garment, the at least one soft-based module configured to monitor a physiological condition through inputs of the physiological measurements, wherein the at least on soft-based module includes at least one sensor configured to make the physiological measurements, characterised in that: the garment comprises complimentary hooks configured to activate the wearable monitoring device responsive to the complimentary hooks making electrical contact.

These and other aspects and features of non-limiting embodiments of the present invention will become apparent to those skilled in the art upon review of the following description of specific non-limiting embodiments of the invention in conjunction with the accompanying drawings.

### BRIEF DESCRIPTION OF THE DRAWINGS

For the purpose of illustrating the invention, the drawings show aspects of one or more embodiments of the invention. However, it should be understood that the present invention is not limited to the precise arrangements and instrumentalities shown in the drawings, wherein:
FIG 1 is an exploded view of an exemplary wearable monitoring device;
FIG. 2 is an exploded view of an exemplary wearable monitoring device;
FIG. 3 is an illustration of a garment integrated with an exemplary wearable monitoring device and various sensors and transceiver;
FIG. 4 is an illustration of an exemplary wearer;
FIG. 5 is an illustration of an exemplary garment;
FIG. 6 is isometric view of an exemplary module;
FIG. 7 is a cross sectional illustration of the exemplary module of FIG. 6;
FIG. 8 is an isometric view of the exemplary module of FIG. 6;
FIG. 9 is an exploded isometric view of an exemplary processing module;
FIG. 10 is an exploded isometric view of an exemplary biometric module;
FIG. 11 is an exploded isometric view of an exemplary power management module;
FIG. 12 is an exploded isometric view of an exemplary interactive In/Out module;
FIG. 13 is an exploded isometric view of an exemplary microfluidic sensor module;
FIG. 14 is an exploded isometric view of an exemplary conductive module;
FIG. 15 is an exploded isometric view of an exemplary mechanical pressure module;
FIG. 16 is an exploded isometric view of an exemplary ultrasound module;
FIGS. 17-23 are schematic illustrations of exemplary garment and module combinations;
FIG. 24 is a schematic illustration of a garment extension attached to another garment at selected anchor points; and
FIG. 25 is a block diagram of a computing system that can be used to implement any one or more of the methodologies disclosed herein and any one or more portions thereof.
The drawings are not necessarily to scale and may be illustrated by phantom lines, diagrammatic representations and fragmentary views. In certain instances, details that are not necessary for an understanding of the embodiments or that render other details difficult to perceive may have been omitted.

Embodiments disclosed herein provide for clothes integrated with a comfortable device to obtain the status of the human body for prevention of cardiovascular diseases by tracking real time information of the heart to help deal with diseases and optimize health by making clothes intelligent and useful.

Prior solutions include devices that can monitor certain body functions worn on the body. Those solutions attempt to address the issues of comfort and durability. Those past solutions include electronics that are bulky, stiff and uncomfortable. Typically, the batteries and microelectronics are stored in a hard case that is unyielding and difficult to integrate into clothing. Those systems are especially not easily integrated into clothing that is worn frequently, such as undergarments.

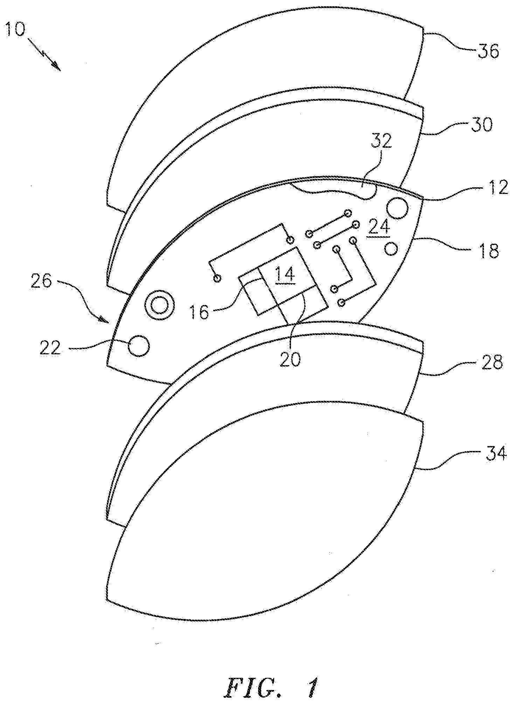

Referring now to Fig. 1, there is illustrated an exploded view of an exemplary wearable monitoring device 10. The wearable monitoring device 10 comprises a circuit board, which may be a printed circuit board 12. The printed circuit board 12 can include a processing unit 14 having at least one electronic component, e.g., microprocessor, 16 built into a substrate 18. A printed circuit board (PCB) mechanically supports and electrically connects electronic components using conductive tracks, pads and other features etched from copper sheets laminated onto a non-conductive substrate. Components (e.g. capacitors, resistors, integrated circuits or active devices) are generally soldered on the PCB. Advanced PCBs may contain components embedded in the substrate. The PCB can include, laminates, copper-clad laminates, resin impregnated B-stage cloth (Pre-preg), and copper foil. The printed circuit board 12 can be made of a flexible material or from a less flexible material or a rigid material. The printed circuit board 12 can include materials such as any kind Rigid PCB with a substrate such as a Flame Retardant, CEM, PTFE; a flex PCB (pyralux, Kapton, copper-clad foil or can be laminated to a thin stiffener) and the combination of both which can be a Rigid Flex. The processing unit 14 can comprise one or more processors 16, a memory 20, and input/output of electric or electronic signals 22. The printed circuit board 12 has a first side 24 and a second side 26 opposite the first side 24.

With continued reference to FIG. 1, processing unit 14 and/or a computing device communicating therewith may be designed and/or configured to perform any method, method step, or sequence of method steps in any embodiment described in this disclosure, in any order and with any degree of repetition. For instance, processing unit 14 and/or a computing device communicating therewith may be configured to perform a single step or sequence repeatedly until a desired or commanded outcome is achieved; repetition of a step or a sequence of steps may be performed iteratively and/or recursively using outputs of previous repetitions as inputs to subsequent repetitions, aggregating inputs and/or outputs of repetitions to produce an aggregate result, reduction or decrement of one or more variables such as global variables, and/or division of a larger processing task into a set of iteratively addressed smaller processing tasks. Processing unit 14 and/or a computing device communicating therewith may perform any step or sequence of steps as described in this disclosure in parallel, such as simultaneously and/or substantially simultaneously performing a step two or more times using two or more parallel threads, processor cores, or the like; division of tasks between parallel threads and/or processes may be performed according to any protocol suitable for division of tasks between iterations. Persons skilled in the art, upon reviewing the entirety of this disclosure, will be aware of various ways in which steps, sequences of steps, processing tasks, and/or data may be subdivided, shared, or otherwise dealt with using iteration, recursion, and/or parallel processing.

The printed circuit board 12 can be sandwiched between a first padding layer 28 coupled to said printed circuit board 12 proximate the first side 24. A second padding layer 30 can be coupled to the printed circuit board 12 proximate the second side 26. The first and second padding layers 28, 30 can be selected from the group consisting of foam, silicon, a material having a cellular structure resistant to electrostatic discharge(ESD) material (i.e., polyurethane), and memory foam, gelatinous material and poly laminate foam that protects the circuit board 12 from water and is comfortable and safe next to a body.

In an alternative embodiment, the printed circuit board 12 can include a thin film cover 32 that envelopes and protects the circuit board 12 from electrostatic discharge and water.

A first protective layer 34 can be coupled, bonded, laminated or layered to the first padding layer 28 opposite the printed circuit board 12. A second protective layer 36 can be coupled, bonded (i.e., tricot bonded), laminated or layered to the second padding layer 30 opposite the flexible printed circuit board 12. The first protective layer 34 and the second protective layer 36 are sealed together and enclose the first and second padding layers 28, 30 and the printed circuit board 12. The first protective layer 34 and the second protective layer 36 can be fabrics (i.e., waterproof polyurethane laminated (PUL) fabrics), soft advanced fabrics, hydrophobic material, and the like.

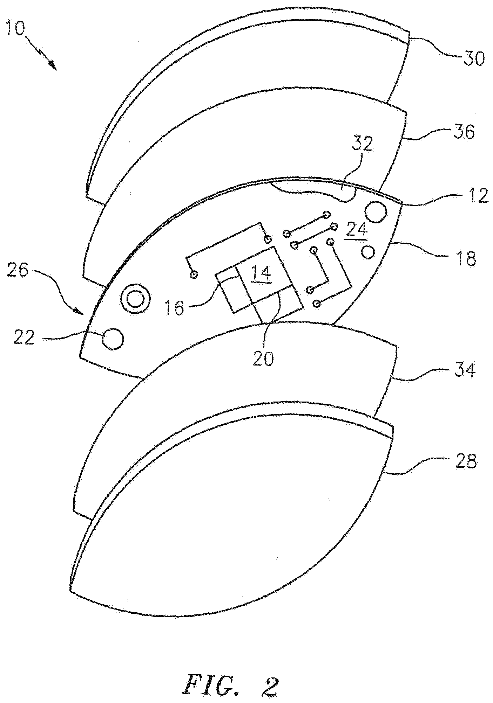

In an alternative embodiment as shown in Fig. 2 the first protective layer 34 and second protective layer 36 can be placed closest to the printed circuit board 12. The first and second padding layers 28, 30 can be placed on against the first and second protective layers 34, 36 opposite the printed circuit board 12 respectively. The first and second protective layers 34, 36, can be coupled to the printed circuit board 12 to seal and protect the printed circuit board 12 from moisture and contaminants.

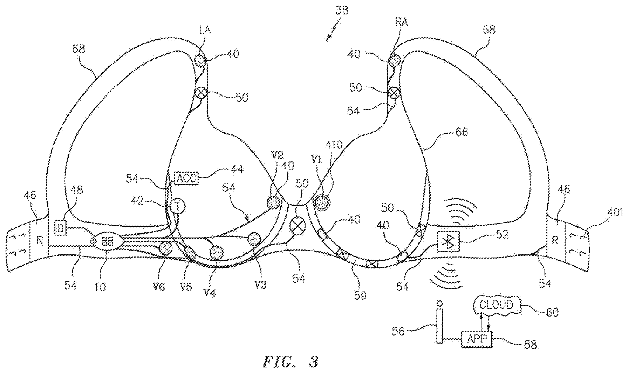

The combination of the protective layers 34, 36 and padding layers 28, 30 surrounding the flexible printed circuit board 12 make up a uniquely wearable monitoring device 10 that can be easily integrated into a garment 38 as shown in Fig. 3.

The garment 38 is shown as an exemplary embodiment, as a bra and can also be any form of garment 38 that is used proximate the body, preferably the torso. Such examples of garments 38 include a brassiere, bustier, bra, corset, babydoll, bralette, basque, bodice torsolette, sports bra, panties, boxers, shirts, pants, jeans, jackets, sweaters, hats, socks and the like.

The garment 38 can include a variety of sensors, it includes at least one soft-based module 40 designed to sense a person's electrophysiology, biological features and the like. The soft-based module includes at least one sensor 40 that can include at least one or more of the following: textile or fiber integrated sensors, acoustic sensors (i.e., Biometric module 190, Ultrasound Module 210), position sensors (i.e., processing module 90), optical sensors (i.e., Interactive I/O Module 144), piezo resistive sensors, temperature sensors (i.e., conductive module 172), electrocardiogram electrodes (i.e., biopotential modules 70), accelerometer (i.e., processing module 90), piezo resistive fabric, microphones (i.e., biometric module 190) and the like. The sensors 40 can be configured to obtain status of the human body for prevention, monitoring and treatment of diseases and health and aging status by tracking real time information of the heart, hereunder at least one or more of the following: heart rate, heart rate variability, heart rate recovery, electrocardiogram (in the following referred to as ECG), heart sound; lungs, hereunder at least one or more of the following: respiratory rate, minute ventilation, maximal oxygen consumption, lungs sounds; body metrics, at least one or more of temperature, movements, position, and the like.

The exemplary embodiment shown at Fig. 3 includes a variety of sensors 40, specifically a temperature sensor 42, an accelerometer 44, a piezo resistive sensor 46, electrophysiological sensors, V1, V2, V3, V4, V5, V6, LA, RA, RL, LL and the like.

A power source 48, such as a battery is shown coupled to the wearable monitoring device 10. The wearable monitoring device 10 can be coupled to the various sensors 40 and power source 48, switches 50, and wireless communicator or transceiver 52 by use of a conductor 54, such as conductive thread, wire and the like.

In addition to the sensors 40, the transceiver 52 can include, Wi-Fi^{™}, BLUETOOTH^{™} wireless communication and/or other RF equipment wirelessly coupled to another transceiver 56, user interface, such as an interface on a computing device such as a smartphone 58, and cloud 60 and has the possibility of connecting to servers, computers, supercomputers or others for AI (artificial intelligence), machine learning and other data processing/interpreting methods. The data collected by the various sensors 40 can be processed by the microprocessor which can perform some low embedded machine learning algorithms 16 and transmitted by the transceiver 52 to transceiver 56 and shared through an application on a computing device and/or smartphone 58, displayed by using algorithms to provide valuable content.

The processing unit 16, the memory 20, the user interface 58, the one or more biometric sensors 40, and the input/output interface or conductive pad 22 may be communicatively connected via communications path(s). It is to be understood that some of these components may also be connected with one another indirectly. In some embodiments, components of FIG. 1 may be implemented as an external component communicatively linked to other internal components. For instance, in one embodiment, the memory 20 may be implemented as a memory on a secondary device such as a computing device and/or smartphone that communicates with the device wirelessly or through wired connection via the I/O interface 22. In another embodiment. The user interface 58 may include some components on the device such as a switch 50, as well as components on a secondary device communicatively linked to the device via the I/O interface/conductive pad 22, such as a touch screen on a smart phone 58, or smart watch and the like. The raw data from the sensors 40 can pass through a processing stage that can include filters, operational and instrumental amplifiers and instrumental, analog to digital converters, analog front end and the like.

In another exemplary embodiment, the sensors 40 can be integrated with a bra wire casing 59. The bra wire casing 59 can include a textile material formed into a tube for receiving a bra wire (not shown) that is utilized to stiffen and support the garment 38. The sensors 40 and casing 59 can be integrated into a unit an integrated with the other sensors 40 of the garment 38.

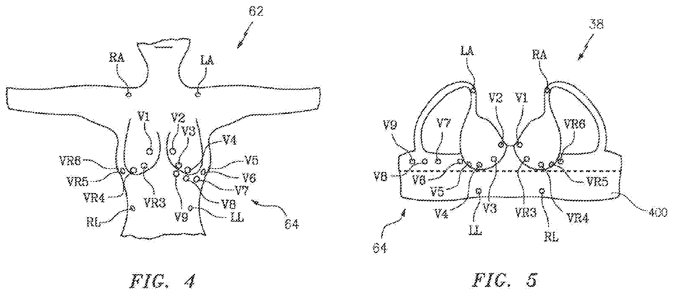

Fig. 4 and Fig. 5 illustrate a wearer 62 and the garment 38 and the variety of sensors 40 placed in locations 64 understood to be optimal for biopotential measurements of the cardiovascular system of the body. For biopotential measurements can be performed, minimum with two or more sensors/modules 40. The first pair of modules 40 will be on the same body axis at symmetrical ends such as LA and RA which are symmetrical or LL and RL are symmetrical too, then every additional biopotential sensor module can be placed individually such as V1-V9 and VR1-VR6 on a different axis of the heart. Similar configurations can be symmetrical to the brain, lungs or other organ or muscle being measured. The American Heart Association (AHA) and International Electrotechnical Commission include electrode positions for electrocardiogram or other cardiovascular measurements. The garment 38 can include sensor 40 locations following these guidelines.

The electrode positions (AHA lead wire labels/ IEC labels, and the drawings are shown with AHA labels).

Electrode positions are commonly known as follows:
RA/R: Middle to outside end of the right clavicle, close to the bone;
LA/L: Middle to outside end of the left clavicle, close to the bone;
RL/N: Lower right trunk, just above the hip;
LL/F: Lower left trunk, just above the hip;
V1/C1: 4^{th} intercostal space at right border of the sternum;
V2/C2: 4^{th} intercostal space at left border of the sternum;
V3/C3: midway between V2 and V4;
V4/C4: 5^{th} intercostal space at midclavicular line;
V5/C5: level with V4 at left anterior axillary line;
V6/C6: level with V4-V5 at left midaxillary line;
V3R/C3R: midway between V1 and V4R;
V4R/C4R: 5^{th} intercostal space, right midclavicular line;
V5R/C5R: level with V4R at right anterior axillary line;
V7/C7: level with V4R at right anterior axillary line;
V8/C8: level with V4-V6 at left midscapular line;
V9/C9: level with V4-V6 at left spinal border.

Different electrode positions can be used to make measurements of the electrocardiogram different leads such as follows:
Bipolar limb leads (frontal plane):
   - Lead 1: RA (-) to LA (+) (Right Left, or lateral).
   - Lead II: RA (-) to LL (+) (Superior Inferior).
   - Lead Ill: LA (-) to LL (+) (Superior Inferior).
Augmented unipolar limb leads (frontal plane):
   - Lead aVR: RA (+) to [LA & LL] (-) (Rightward).
   - Lead aVL: LA (+) to [RA & LL] (-) (Leftward).
   - Lead aVF: LL (+) to [RA & LA] (-) (Inferior).
Unipolar(+) chest leads (horizontal plane):
   - Leads V1, V2, and V3: (Posterior Anterior).
   - Leads V4, VS, and V6: (Right Left, or lateral.

The wearable monitoring device 10 can be inserted in the brassier in a cup 66, the device 10 can be removable (from a pocket of thin textile) and/or sewn or embedded in the cup 66 itself, options will be available, the first and second protective layer 34, 36 can be replaced by a similar feeling textile with the device 10 inside, connected to two electrodes 40 in diverse positions. In another exemplary embodiment, the wearable monitoring device 10 can comprise the entire cup 66 as a single unit. The entire cup 66 with device 10 can be removable, integrated or a stand-alone unit.

There are many configurations of single-lead electrodes near the chest area, that covers all the positions of the recent ecg patches can be placed at (such as delta epatch, mc10, and other patches for arrythmias) the wearable monitoring device can have positions as above and can be inserted in the brassiere as part of the garment 38.

This monitoring device can be used for the inner-lining of padded bras and as cup input, replacement or complement, its padding component can come in many ranges of thickness, if padding is thick enough the flexible printed circuit board can be changed for a regular printed circuit board, as long as it is still not noticeable to the user and comfortable. A Single-lead configuration can include any two electrodes in different electrode positions (i.e., LA and RA).

In an alternative embodiment, the flexible circuit board 12 can be located under the arm, on the side of the bra 38 (left or right), or in the cup 66 or in the back. The flexible circuit board 12 is removable from a pocket and/or sewn. Several multiple lead configurations are available, including some or all of the lead-placements (also could be referred to as the combination of two or more diverse single-lead positions mentioned above).

In an alternative embodiment, the switches 50 can be configured as mechanical push button, touch sensor, and the like (round or square or other shape) and placed in 1, 2 and/or 3 strategic places:
a. 1 switch 50: in the mid-center of the bra 38 or on the left or right side of a bra strap 68 and the cup 66.
b. 2 switches 50: mid-center of the bra 38 and either the left or right side of the bra strap 68 and the cup 66 or left and right sides of the bra strap 68 and the cup 66.
c. 3 switches 50: mid-center of the bra 38 and left and right sides of the bra strap 68 and the cup 66.

In an alternative embodiment, the multiple switches 50 can be utilized as follows:

When in a 24-hour ECG Monitoring process (continuous monitoring): when the switch 50 is pushed by the wearer the device 10 stores the set of data in special format (flags/tags) the next flow of data from the heart and other sensors 40 for a time frame of at least 30 seconds to maximum 10 minutes depending on wearer's preferences, therefore if they feel a symptom or need to share a specific moment the data is tagged by use of the switch 50 and saved as priority automatically after pushing the switch 50 without opening an application or the need of any additional devices beyond the bra 38 itself.

Resting ECG: The wearer 62 lies down or gets into a resting position (i.e., seated) and pushes the switch 50 while a recording is made for a time frame of at least 30 seconds to maximum 2 minutes, depending on wearer's profile preferences.

Stress ECG: The user exercises either on a treadmill machine or bicycle and pushes the switch 50 and records for a time frame of at least 30 seconds to maximum 30 minutes depending on user preferences.

Event ECG: During non-continuous monitoring, the wearer 62 presses a direct record switch 50 and the device 10 records and stores the heart's electrical activity taking events at different moments at any part of the wearer's day for a time frame of at least 30 seconds to maximum 2 minutes depending on preferences and quantity of pushes during an event.

Event Recorder + Direct communication: During non-continuous monitoring, the wearer 62 presses a direct record switch and the device 10 records and stores the heart's electrical activity. The information can be sent to the physician or caregiver over the cloud 60 immediately.

The wearable monitoring device 10 can be utilized to obtain status of the human body for prevention and monitoring of cardiovascular diseases by tracking real time information of the heart, hereunder at least one or more of the following: heart rate, heart rate variability, heart rate recovery, electrocardiogram (in the following referred to as ECG), heart sound; lungs, hereunder at least one or more of the following: respiratory rate, minute ventilation, maximal oxygen consumption, lungs sounds; body metrics, hereunder at least one or more of the following: temperature, movements, position.

The wearable monitoring device 10 is configured to improve the measurements of the human body done through sensors, hereunder at least one or more of the following: textile or fabrics integrated sensors, acoustic sensors, position sensors, optical sensors, piezo resistive sensor, temperature sensor. The sensors are connected to protected circuits hereunder at least one or more of the following: protected circuit boards, flexible, semi rigid or rigid printed circuit boards. The sensors and the protected circuits will be placed seamlessly within clothes.

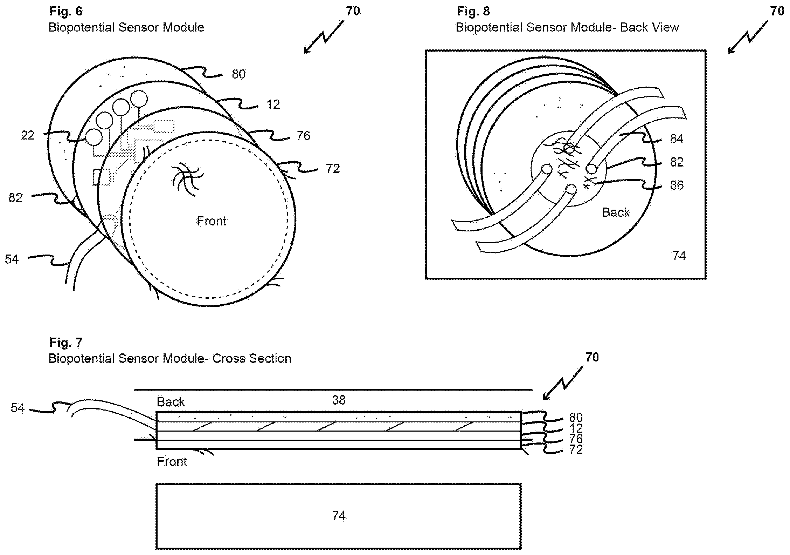

Referring to Figs. 6, 7 and 8 an exemplary bio potential sensor module 70 is shown. The padding layer 80 can have similar properties to the above disclosed padding layers 28, 30. The padding layer 80 can include materials such as, foam, gelatinous material, silicon, and polylaminate foam. The padding layer 80 can also be layer 30 and 36 already coupled to each other, similarly to layer 28 and layer 24 already coupled to each other. Front of padding layer 80 is the printed circuit board 12 and back of padding layer 80 is garment 38. The back of padding layer 80 is configured to be attached directly to the garment 38 (see figure 3). The back of padding layer 80 can be a fabric, cloth material that can receive stitching, adhesives, and the like for attachment to the garment 38. As disclosed in detail above, the garment 38 can include but is not limited to an extension of a bra, interior of a tee shirt, a sock, underwear, hat, head band, and the like. In an exemplary embodiment of the sensor module 70, the interior of the garment 38 is needed to support the sensor module 70 so that the front 72 can contact the skin of the wearer 62. The bio potential sensor module 70 includes a fabric layer 72 which is configured to directly contact the skin 74. The fabric layer 72 is conductive can be hydrophobic and can have a tenacious grip to the skin 74. Conductive fabric layer 72 may function as a conductive surface contacting the skin. A conductive tape, or doubled sided adhesive 76 configured to conduct data sensed in the skin 74. A printed circuit board, flexible printed circuit board, thin film with conductive material, textile with conductive ink or similar or simply circuit layer 12 is coupled to the conductive tape 76, Circuit layer 12 has a conductive pad 22 to connect through the conductive adhesive 76 to the conductive fabric 72 to the skin 74. The conductive pad 22 in circuit layer 12 is important for the right signal acquisition from the body, therefore the pad is of highly conductive material (i.e., Ag/AgCl, immersion Gold, Silver or similar conductivity) should at least be sized enough to solder or connect to a wire, in other words the conductive pad 22 width: > 150% of wire 54 diameter. The smaller this conductive pad 22 the bigger the conductive fabric size in layer 72 can be. The conductive pad can also be wireless, requiring the pad to be bigger size, the pad can have any shape any combinations of size and shape can be used as long as they have the proportional distribution that conductive fabric size area in layer 72 is much bigger when conductive pad 22 size area in layer 12 is smaller and the bigger the conductive pad 22 area in layer 12, the smaller the conductive fabric area size can be in layer 72. With the smallest size of the conductive pad 22 being that of a wire pad.

A padding layer 80, which can consist of a combination of padding layers 30 and 28 and protective layers 36 and 34, covers the printed circuit board 12 opposite the conductive tape 76. An opening 82 can be formed in the padding layer 80 to provide access for conductive leads or wires, connectors 54 to couple with layer 12, 78 through the padding layer 80. A sealing material can be utilized to seal the padding layer 80 around the conductive leads, wires, connectors 54 to protect layer 12, 78. The sealing material can be waterproof. Although the bio potential sensor module 70 is shown with conductive leads 54 configured to transmit electrical signals from the layer 12, 78, a wireless transmitter can be utilized instead of the conductive leads 54. Although opening 82 is shown, there can be no opening and the sealing is through lamination or similar and sealing material 54 can be on top of layer 80.

As seen in Fig. 8, module 70, biopotential modules can be active or inactive. Active means that layer 12, 78 can consist of a pcb (printed circuit board, flexible printed circuit board, fabric with conductive ink, thin film and the like) with electrical components such as an integrated circuits, operational amplifier, resistors, capacitors and similar filter and amplifier configurations on the side that is next to layer 80, this side would have a wire pad 54 to connect the output to other modules or would transmit this output wirelessly, by adding an rf antenna or similar and microcontroller, it can also have two additional wires 54 for power and ground sources or have a small battery prior to layer 80 for wireless. Inactive biopotential modules would not have the aforementioned electrical components, it would only have a wire pad 54 on either side of the layer, besides the larger conductive pad 22 that is next to layer 76 and connects to conductive fabric 72, if this fabric is bigger sized, then the wire pad would be only the wire soldered, attached or coupled to the side, else if the conductive fabric is more similar size to the pad size, the wire can be on a connected pad on the other side of the layer 12, 78, next to layer 76. If the inactive module is wireless, the side of 12, 78 next to layer 80 will have an rf antenna, microcontroller and power source.

The biopotential sensor module 70 usually is sewn, glued, attached in the garments in pairs, which means that they are placed in symmetrical positions of the body (i.e.; left and right arms, left and right side of the neck, legs, top or bottom, and the like). Together they measure biopotential differences. An individual biopotential sensor can be used when there is at least one or more pairs are already placed, therefore the third single 70 module can measure the biopotential differences with the result obtained by the other multiple 70 sensors. To obtain such measurements from the body, in mV or other unit, the embodiment needs to be touching the skin 74, this means the garment needs to be tight enough so that the module touches the skin 74. One side (the non-conductive side) 80 of the module is sewn, stitched, glued, attached or similar in the inner side of the garment and the other side (conductive side) 72 of the module will be touching the skin when the garment is worn by the wearer 62. The biopotential modules can do measurements of the body such as plethysmography, electrocardiography (ECG), impedance cardiography (ICG), Electroencephalogram (EEG), Electromyography (EMG), impedance, resistance and potential differences and others.

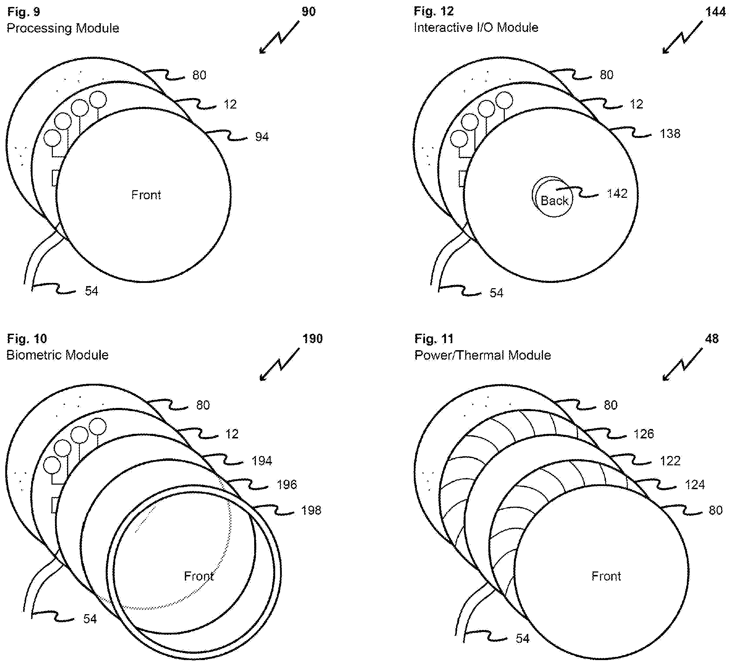

Referring to Fig. 9 an exemplary processing module 90 is shown. The processing module 90 can include an integrated circuit like a microcontroller, accelerometer, memory, application specific integrated circuit (ASIC), analog front end (AFE) or other in layer 12 between a front layer 94 and back layer 96. A conductor 54 can be coupled to the processor layer 92 configured to transmit signals, power and the like. The processor module 90 can be configured to store data, send and receive data from sensor modules, quickly analyze data in real-time and transmit information regarding the wearer 62. Understand the wearer 62 posture, movement, resting, activity, fall detection and similar analysis with their coordinates according to the location where placed on the body as well like the back, front or any of their joints. Other embodiments of this module are described above.

Figure 10 exemplary textile-based biometric sensor module 190. The biometric sensor module 190 can include a front layer 198 that can be waterproof. The front layer 198 is configured to touch the wearer's skin 74. The front layer 198 can be made of any heat dissipating material that can be shaped into a ring such as rubber or be textile-based to function as an acoustic diaphragm that can provide an auscultation area that connects to the second layer 196.

A second layer can be a membrane 196. The membrane layer 196 can be configured to absorb sound pressure waves originating in the body. The membrane layer 196 is mechanically coupled to the acoustic stethoscope layer 194. In an exemplary embodiment, the acoustic stethoscope layer 194 may be a stamped cavity made of metal or flexible conductive material, or soft or hard, reflective, nonporous material. The acoustic stethoscope could also be an ultrasonic sensor that absorbs sound pressure waves originating in the body. The acoustic stethoscope layer 194 can be configured to couple to a printed circuit board layer 12 and can include a wire or cable 54, printed ink, conductive thread and the like. The second side 112 opposite the first side 110 can receive an adhesive (not shown). A connector 54 can be coupled to the circuit 12.

A padded protective layer 80 can be coupled to the circuit layer 12 opposite the acoustic stethoscope layer 194. The back padding layer is configured to next in the lining of a garment such as 38. Data from this module is digitized and can be viewed as a phonogram that is obtained through soft-based sensors 40.

Figure 11 shows an exemplary power or thermal module 48. The power management module 48 includes a battery and/or circuit layer 12 nested between a first layer that can be a heat sink 124 to dissipate or holds heat and second layer can also be a heat sink 126 to dissipate or hold heat. A front padding layer 80 and back padding layer 80 are located on either side of each of the heat sink layers 124, 126 sandwiching the front and back of the power module 48. The protective layers 80 can include a moisture or water resistant material that conforms to the power module 48 contours. In an exemplary embodiment, the protective layers 80, can include a shrink wrap material that can tightly conform with the contours and seal out moisture, water and contaminants. A connector or wire 54 can be coupled to the battery/circuit layer 122. The connector 54 can be utilized to provide electrical power to other modules in the garment 38 when the other modules don't have their own battery. This module can also be used to pull heat away from wearer 62 through layer 80 touching the skin or pull heat to wearer 62 through layer 80 by integrating the soft module via sleeves, socks or around the torso.

Figure 12 shows an exemplary interactive I/O module 144. The interactive I/O module 144, can be utilized as a switch or button, light emitting diode (LED) or photo plethysmography (PPG) or other optical sensor. The interactive I/O module 144 can include a back layer 138 of fabric, foam, gelatinous material, silicon and/or polylaminate foam configured to receive a switch, button and/or LED and/or optical sensor from 142. 142 can also be an input/output interface coupled to a circuit layer 12. In this module, back layer 80 is configured similarly to the front layer 138. The back layer 80 is configured to attach to the garment 38. A connector 54 can be coupled to the circuit layer 12 and provide connectivity to other modules on the garment 38. This module can be both in the inner side or outer side of the garment 38.

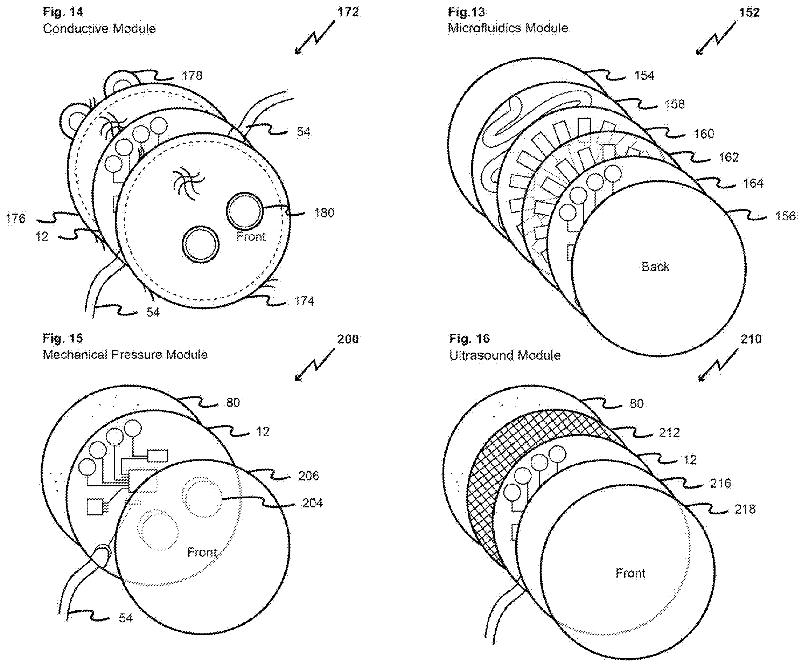

Figure 13 shows an exemplary textiles-based microfluidic sensor module 152. The microfluidic sensor module 152 can include a front layer 154 of a foam, gelatinous material, silicon and/or polylaminate foam that is configured to contact the wearer's skin 74. The front layer 154 can be configured to attach to a one-way valve 158 to extract necessary fluids from the wearer's body. In an exemplary embodiment, the valve 158 can include a stainless steel, silicon and/or polylaminate foam. The valve 158 is configured to attach to a fluid tray 160. The fluid tray 160 is configured to store extracted body fluid for testing. In an exemplary embodiment, the fluid tray 160 can include soft, bendable materials or acrylic, glass, or similar material with an engraved path for fluid dynamics. The fluid tray 160 is configured so that it can eject from the module and be replaced and replaced with a new fluid tray that attaches to an air-channel 162. The air channel 162 is configured to store air that will allow body fluid stored in the fluid tray 160 to flow appropriately to the corners of the fluid tray 160 and be tested for health data interpretation. In an exemplary embodiment, the air channel 162 can include soft, bendable, silicon, acrylic, glass or similar material and precious metals and soft metals engraved with a mirrored path as the one in the fluid tray 160. The air channel 162 is configured to attach to a circuit layer 12. The circuit layer 12 is configured to interpret the health data from the air channel 162. A protective back layer 156 is configured to connect to the circuit layer 12, 164. A connector 54 can be coupled to the circuit layer 12 and provide connectivity to other modules on the garment 38. The shown microfluidic sensor module 152 can be detached from garments 38 and similar. Fluids that come from wearer 62 and go through this module 152 through the ejectable soft cartridges 162, 160 and 158 can include blood, urine, sweat and saliva. Garments that can have this modules include but are not limited to underwear, bra, t-shirts, socks, long-sleeves, gloves and forehead bands.

Figure 14 shows an exemplary textile-based conductive sensor module 172. The conductive sensor module 172 can include a front layer 180 that is configured to contact the wearer's skin 74. In an exemplary embodiment the front layer 180 is made of a conductive textile 72, conductive non-allergenic materials or metal snap 180 that can consist of steel, Silver plated, high conductivity, Tin/copper coated, Cobalt alloy top coating, Silver (Ag) plated, Ag/AgCI silver/silver chloride. The metal snap can have single, double or triple or more snap-style pellet configuration with fabric separation in between or have mix of conductive fabric with non-conductive fabric in between. The front layer 180 is adhered to a textile layer 174. In an exemplary embodiment the textile layer 174 can be made of organic or synthetic textile that is non-conductive. The textile layer 174 is coupled to a sealed, flexible printed circuit board layer, thin film or printed circuit board layer 12. The sealed 12is coupled to another textile layer 176. In an exemplary embodiment, this second textile layer 176 is similar to layer 174 in that it can be made of organic or synthetic non-conductive textile. The back layer 178 can be the complementary half of the front layer 180, this module can also have 178 between circuit layer 12 and layer 176 and in another embodiment may not have layer 178, making 176 the back layer. In an exemplary embodiment the back layer can consist of the female or male half of the snap that completes the front layer 180. The textile layers 174 and 176 can be used to adhere the module to the garment by sewing, bonding or similar apparel manufacturing techniques.

Layer 182 is a flexible pcb, thin film, fabric with conductive printed ink, silver, gold, copper or the like where one side has the adhesive to the front layer and the other side has wires, cables, printed ink fabric, conductive threads or other type of connectors that will be longer and connect to another module such as the processing module or can be wireless and transmit the data captured. This layer can also have other integrated circuits or passive components soldered, sewn or glued.

Layer 176 can be a padding layer consisting of foam, gelatinous material, silicon and polylaminate foam.

Layer 176 can also be a combination of padding and fabric or just a fabric, cloth, sewable or glueable material that will be attached to the garment, such as an extension of a bra, interior of a t-shirt, interior of a sock, interior of underwear, hats and the like. This can only be placed in the interior of the garment.

Figure 15 shows an exemplary mechanical pressure sensor module 200. The mechanical pressure sensor module 200 can include a front layer 206. In an exemplary embodiment the front layer 206 can be made of a perforated siliconized textile piezo-resistive fabric, a substrate or similar. The front layer is configured to adhere to the wearer's skin 74. On the opposite side, the front layer is configured to a piezoelectric layer 204. The piezoelectric layer 204 can be configured to send to and absorb sound pressure waves from the wearer 62. In an exemplary embodiment, the piezoelectric layer can be made of a substrate with polyamide, cu electrode, Cu/Sn electrode, piezo pillars, and epoxy filling and similar. The piezoelectric layer 204 is coupled to the printed circuit layer 12. The printed circuit layer 12 can have a wire 54 that connects the mechanical pressure module 200 to other modules 40 in the garment 38. The printed circuit layer is coupled on the other side to a back layer 80.

Figure 16 shows an exemplary ultrasound sensor module 210. The ultrasound sensor module 210 can include a front layer 218. The front layer 218 is configured to adhere to the wearer's skin 74. In an exemplary embodiment, the front layer 218 can be made of a protective polymer or similar that is configured as an acoustic lens. The front layer 218 is coupled to a second, similar acoustic layer 216. The second acoustic layer is coupled with a printed circuit 12 that can have a connecting wire 54. The printed circuit 12 is coupled to a backing or acoustic insulating layer 212. In exemplary embodiment, the acoustic insulating layer 212 can be made of plastic, rubber, silicon or other materials to control acoustic attenuation. The acoustic insulating layer 212 is configured to adhere to a back layer 80 that nests within the lining of garment 38.

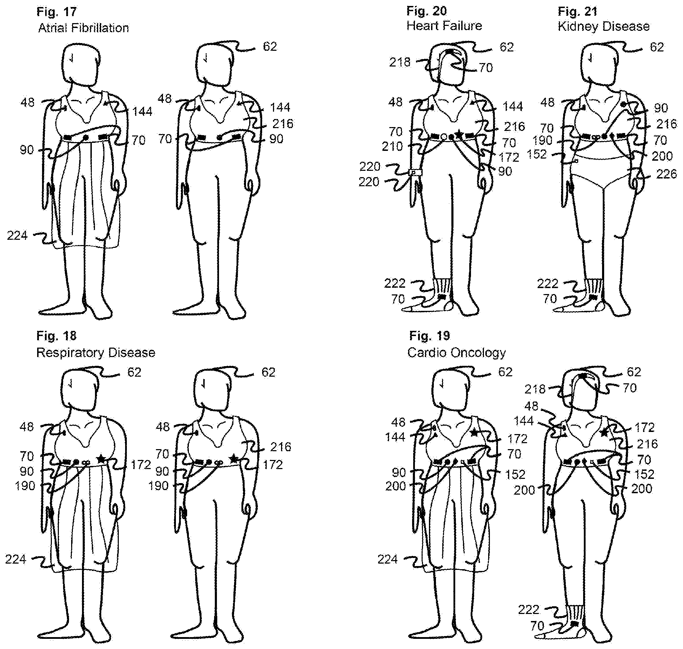

Referring also to Figures 17-23 schematic illustrations of exemplary garment and module combinations are shown. The exemplary garment and module combinations are for disclosure purposes, so it is contemplated that other combinations of modules and garments can be obtained within the scope of the appended claims. It is contemplated that various combinations of modules disclosed herein can be arranged on garments and located at beneficial positions relative to the wearer so as to provide optimal monitoring of the wearer for a host of symptoms and diseases and chronic conditions such as, but not limited to, epilepsy, Alzheimer's, hormone therapy, obesity, type 1 diabetes, type 2 diabetes, smoking, hypertension, genetic predisposal to heart disease or heart conditions, aging-related diseases and conditions, endometriosis, septicemia, depression, pulmonary embolism, ischemia, sleep apnea etc. Module combinations in exemplary garments can also be used to screen for the latter diseases and conditions.

The Atrial Fibrillation (Afib) illustration at Figure 17, shows a wearer 62 with a garment 38 equipped with a variety of sensors 40 to detect, monitor, treat and manage Afib or stroke recurrence prevention via data from the sensor modules that get sent to a computing device and/or phone, and analyzed and can be provided to physicians and wearers. The sensors 40 can be coupled to the garment 38 based on a predetermined disease to provide input/output about the condition of the wearer 62. In the exemplary embodiment at Fig. 17, the garment includes at least two sensors 40. At least a pair of Bio-potential sensor modules 70 and one processing module 90. Other sensors can be added for additional monitoring of Afib and stroke prevention such as mechanical pressure 200, ultrasound modules 210, micro-fluidics module 152, interactive I/O modules 144, and power modules 48 can cooperate to provide more information for longer periods of time about the wearer 62. The Fig. 17 shows both a night gown 224 (e.g. night dress, night shirt, pajama top, tank top, tank top with sewn in chest support etc.) and a bra 216 (e.g. contour bras, wireless bras, t-shirt bras, push-up bras, demi and balconette bras, bralettes, convertible bras, strapless bras, sports bras, minimizer bras, stick-on bras, backless bras, surgical bras, nursing bras, bikini tops, training bras, swimsuits, bodys, shapewear and corsets) as examples.

Measurements performed by these modules include providing long-term monitoring capabilities of the wearer 62 can include but are not limited to asymptomatic and symptomatic electrocardiogram abnormality detection, continuous and event monitoring, weakness and fatigue, reduced ability to move or exercise, lightheadedness, dizziness, shortness of breath and respiration rate, chest pressure, pain or discomfort, stress or electrodermal response changes, blood tests, troponin tests. This also works well with the information of the wearer 62 stored in cloud 60 and the app 58 to provide an enhanced view and generate automated reports for the physician of the trends, changes and treatment impact of the wearer 62.

Figure 20 shows a wearer 62 with garment 38 having a combination of predetermined sensors 40. To screen for, detect, manage and treat digitally the ailments of Heart Failure. Wearers 62 are patients and physicians can collect data that can be further analyzed with algorithms from 2 or more modules depending on the level of risk or diagnosis for heart failure. In the exemplary embodiment of Fig. 17c data for heart failure can be collected from at least the following sensors 40: more than 3 Bio-potential sensor modules 70, processing modules 90 and may additionally have power module 48, biometric modules 190, micro-fluidics module 152, conductive modules 172, ultra-sound modules 210 and interactive I/O modules 144 depending on the patient's needs. In an exemplary embodiment, up to 4 garments with sensors 40 can capture valuable data between day and night for heart failure patients or people at risk of heart failure, these garments can be worn as needed and can include underwear 226 (e.g. boy shorts, classic briefs, hipsters, thongs, French-cut panties, G-string, shapewear, control briefs, seamless, Brazilian brief, leak-proof underwear, cheeky underwear, high-cut briefs, jock straps, trunks, boxers, mid-way trunks etc.), pants, leggings, sleeves, arm tights, leg tights, shirt, bra 216, headband 218 (i.e. bands to hold hair down, as jewelry, or medical bands), wrist band 220 (e.g. watch, bracelet, wrist band, cuff etc.) and sock 222 (e.g. compression socks, no-show socks, liner socks, low-cut socks, athletic socks, quarter anklets, over-the-calf socks, mid-calf socks, pantyhose, toe covers, toe socks, toe shoes etc.). Measurements performed by these modules include providing long-term monitoring capabilities of the wearers 62 can include but are not limited to sudden weight gain, electrocardiogram, reduced ability to move or exercise, mental confusion, ankle swelling, abdominal pain, shortness of breath, increased fatigue, loss of appetite, coughing and wheezing, fluid buildup, blood tests, troponin tests, echocardiogram, hydration level, stress tests and others for corrective interventions, treatments and early detection as well as improved quality of life by understanding their disease stages better from day to day, minute to minute, second to second data collection.

Figure 18 shows a wearer 62 with garment 38 having a combination of predetermined sensors 40. To screen for, detect, manage and treat digitally Respiratory Diseases, patients and physicians need to collect data from at least 1 processing module 90. Additional modules, depending on the level of risk for Respiratory Disease can be included. In the exemplary embodiment of Fig. 18 data for Respiratory Disease can be collected from the following sensors 40: Bio-potential sensor modules 70, conductive modules 172, biometric modules 190, processing modules 90, power module 48, ultrasound modules 210 and interactive I/O modules 144. In an exemplary embodiment, garments that capture the required data between day and night and as needed can include: the daily bra 216, and night dress/shirt 224. Measurements performed by these modules include providing short and long-term monitoring capabilities of the wearers 62 can include but are not limited to symptom tracking via impedance measurements, body temperature for cold, fever or flu, continuous and event electrocardiogram monitoring, weakness and fatigue, reduced ability to move or exercise, lightheadedness, dizziness, shortness of breath and respiration rate, congestive, phlegm discomfort, blood tests, spirometry tests, minute ventilation, maximal oxygen consumption, lungs sounds. This also works well with the information of the wearer 62 stored in cloud 60 and the app 58 to provide an enhanced view and generate automated reports for the physician of the trends, changes and treatment impact of the wearer 62.

Figure 21 shows a wearer 62 with garment 38 having a combination of predetermined sensors 40. To screen for, and detect, manage and digitally treat Kidney Disease, patients and physicians need to collect data from at least a pair of biopotential modules 70 and more modules can be added depending on the level of risk for Kidney Disease. In the exemplary embodiment of Fig. 21 data for Kidney Disease can be collected from the following sensors 40: Bio-potential sensor modules 70, biometric modules 190, processing modules 90, power module 48, micro-fluidics module 152, conductive modules 172, mechanical-pressure module 172 and interactive I/O modules 144. In an exemplary embodiment, garments that capture the required data between day and night and as needed can include: the daily bra 216, underwear 226 and socks 222. Measurements performed by these modules include providing short and long-term monitoring capabilities of the wearers 62 can include but are not limited to mental sharpness, electrocardiograms, nausea, muscle twitching and cramps, vomiting, hydration level, swelling feet, fatigue and weakness, itching or allergies, fluid build-up, sleep disturbances and tracking, blood tests, urine tests and blood pressure. This also works well with the information of the wearer 62 stored in cloud 60 and the app 58 to provide an enhanced view and generate automated reports for the physician of the trends, changes and treatment impact of the wearer 62..

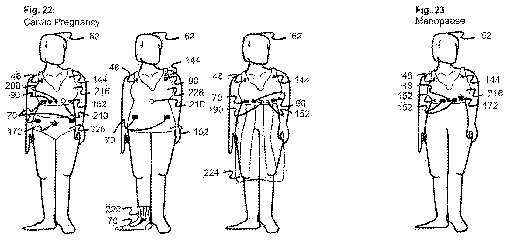

Figure 23 shows a wearer 62 with garment 38 having a combination of predetermined sensors 40. To screen for, detect, control and manage menopause, reproductive cycles, fertility tracking and diseases like endometriosis wearers 62 will benefit from the data collected from at least the conductive module 172 with core body temperature or basal temperature sensing capabilities and more modules can be used depending on the stage for menopause or any other needs from the reproductive system. In the exemplary embodiment of Fig. 23 data for menopause, menarche, peri- and post-menopausal, reproductive cycle and can be collected or outputs to the wearer 62 from the following sensors 40: Bio-potential sensor modules 70, processing modules 90, thermal module 48, biometric modules 190, micro-fluidics module 152, ultrasound modules 210 and interactive I/O modules 144. In an exemplary embodiment, garments that capture the required data between day and night and as needed can include the daily bra 216.

Measurements performed by these modules include providing short and long-term monitoring capabilities of the wearers 62 can include but are not limited to hot flashes, electrocardiograms, night sweats, period cycle tracking, fertility window tracking, follicle-stimulating hormone test, other hormone tests, urine tests, gonadotropin test, muscle twitching and cramps, vomiting, hydration level, swelling feet, fatigue and weakness, pregnancy tests, fluid build-up, sleep disturbances and tracking and blood pressure. This also works well with the information of the wearer 62 stored in cloud 60 and the app 58 to provide an enhanced view and generate automated reports of the trends, changes and treatment impact of the wearer 62.

Figure 19 shows a wearer 62 with garment 38 having a combination of predetermined sensors 40. To screen for, detect, diagnose control, manage and digitally treat oncology early detection, post-diagnosis or post-treatment and during remission of cancer and cardio-oncology to ensure the wearer 62 cardiovascular system of cancer patients and survivors that can have potential risk of developing heart conditions if patients take certain types of cancer drugs, or following radiation treatment to the chest. The wearer 62 will benefit from the data collected from at least a pair of bio potential sensor modules 70 with electrocardiogram sensing capabilities and more modules can be used depending on the risk level of the oncology or cardio-oncology related disease. In the exemplary embodiment of Fig. 19 data biological, physiological and other data can be collected from the following sensors 40: Bio-potential sensor modules 70, processing modules 90, power module 48, mechanical pressure modules 200, micro-fluidics module 152, conductive modules 172 and interactive I/O modules 144. In an exemplary embodiment, garments that capture the required data between day and night and as needed can include the daily bra 216, a night dress/shirt 224, a headband 218, and socks 222.

Measurements performed by these modules include providing short and long-term monitoring capabilities of the wearers 62 can include but are not limited to blood pressure, mental confusion, sudden weight gain, electrocardiogram, ankle swelling, abdominal pain, fever or headache, shortness of breath, increased fatigue, loss of appetite, coughing and wheezing, fluid build-up, insulin blood test, cholesterol blood test, other blood tests, ultrasound, heart imagine, blood clot detection, CT/MRI scan, echocardiogram, stress and hydration levels, cardiac toxic side effects from oncology treatment. This also works well with the information of the wearer 62 stored in cloud 60 and the app 58 to provide an enhanced view and generate automated reports for the physician of the trends, changes and treatment impact of the wearer 62.

Figure 22 shows a wearer 62 with garment 38 having a combination of predetermined sensors 40. To screen for, detect, diagnose, control, manage and digitally treat cardio pregnancy, any risk of cardiovascular disease, blood clots or artery dissection or bursts while pregnant, or female reproductive health related diseases such as hyperlipidemia, gestational diabetes, preeclampsia and polycystic syndrome or hemorrhages. This monitoring device would serve to assess the wearer's 62 cardiovascular system does or does not have a potential risk of developing heart conditions upon ingesting certain drugs, or following body stresses or alternative treatments. The wearer 62 will benefit from the data collected from at least a pair of bio potential sensor modules 70 with electrocardiogram sensing capabilities and more modules depending on the risk level of the cardio-pregnancy or female reproductive system related disease. In the exemplary embodiment of Fig. 22 biological, physiological and other data can be collected from the following sensors 40: Bio-potential sensor modules 70, processing modules 90, power module 48, mechanical pressure modules 200, micro-fluidics module 152, conductive modules 172, biometric modules 190, ultrasound modules 210 and interactive I/O modules 144. In an exemplary embodiment, garments that capture the required data between day and night and as needed can include the daily bra 216, a night dress/shirt 224, a headband 218, a camisole 228 (e.g. camisole, shapewear, maternity belt, maternity band, belly band etc.) socks 222.

Measurements performed by these modules include providing short and long-term monitoring capabilities of the wearers 62 can include but are not limited to temperature changes, electrocardiograms for wearer 62 and electrocardiograms for fetus if she is pregnant, night sweats, period cycle tracking, weight gain, activity levels, cholesterol blood tests, triglycerides blood test, muscle twitching and cramps, vomiting, hydration level, swelling feet and ankles, fatigue and weakness, itching or allergies, fluid build-up, sleep disturbances and tracking, blood tests, anemia test, urine tests, Doppler ultrasound, snoring, insulin blood test, headaches, abnormal swelling in body parts including hands and face, electroencephalogram, fetal heart rate, fluid in the lungs, blood pressure, baby movement, baby activity, baby vital signs, contractions, monitoring repercussions of miscarriages, pregnancy stress on the organs, respiratory system, digestive tract, muscular system such as her abdominal, exercise response while pregnant, heart rate, heart rate variability. This also works well with the information of the wearer 62 stored in cloud 60 and the app 58 to provide an enhanced view and generate automated reports for the physician of the trends, changes and treatment impact of the wearer 62.

The advantages of the wearable monitoring device is to configure a bra with sensors and washable circuits, because it is located in the critical anatomical sections of the body allowing for monitoring of the heart, lungs and more. The bra will monitor critical health parameters of women, empowering them by providing information critical to their bodies. Since bras are worn daily, the wearable monitoring device will deliver valuable and meaningful, out of the care provider's office continuous and/or event based information of cardiac health, respiratory health and more, in a seamless and safe way.

Another advantage of the wearable monitoring device is that it comprises washable flexible padded encasing for circuits to be used in daily clothing, being an unnoticeable additional material attached to the daily clothing and keeping the comfort and utility of the daily clothing, by being seamless and not interfering with how the clothing is normally used.

The wearable monitoring device may be inserted into, removed from and sewn into a plurality of compatible garments (e.g., bralletes, brassieres, camisole tops). It can be easily integrated as padding, as the cup or a clothing component, sewn or removable, in garments. The device integrates into textiles, fabrics and clothing, it is soft flexible insertable and sewn, leaving behind the use of hard, bulky devices that are attached separately to garments.

This advanced device can be a cup of a bra, which is the fabric covering the breast of women. It can be integrated in a smooth way that is not noticeable to the user in a variety of bra types and coverage levels such as full coverage, a percentage of coverage (medium coverage), demi or balconette or others such as almond shape, eye, and or triangular shaped cups and even a flat squared or round shape.

This monitoring device can be used for the inner-lining of padded bras and as cup input, replacement or complement, its padding component can come in many ranges of thickness, if padding is thick enough the flexible printed circuit board can be changed for a regular printed circuit board, as long as it is still not noticeable to the user and comfortable.

The monitoring device has been designed to improve signal-to-noise ratio and reduce instrumental noise, acquire better signals from the body in regards to the sensor being used. Each module includes layers that increase their capacity to sense the physiological parameters to provide best data from each sensor type for data-driven monitoring and treatment of patients with different diseases and/or health states, where a "health state" is defined for the purposes of this disclosure as a physiological state of a user that may include a disease state, a precursor state for a disease state, or a status of the user's constitution with regard to likelihood of entering a given disease state. The devices include locations, materials and components to integrate seamlessly into an everyday garment so that noise is reduced and signal acquisition increased. Access to enable unique user experience by allocating the sensors in a place where it is comfortable for each unique body size, shape and form, obtains key parameters for their different diseases or health status and is invisible to their daily interaction with the garment, so no habit changes. Modular capacity of adding or removing modules into the garments depending on the long-term or short-term monitoring, treatment, disease diagnosis, detection and tracking is an advantage to tailor treatment and make it personal to each individual's needs and learning, via algorithms using the data collected from their body. Also the modular design aims to provide longer life cycle of the garment with wearable monitoring capabilities, as each module can have its own battery and be wireless or be interconnected and have optimal power management. It also enables reduction of electronic waste in smart garments by using layers that can be reused, replaced and transferred to other garments.

The monitoring device can have the shape of an adjustable extension 400 for a bra or a sensor fabric swatch for other garments 38 comprised of fabrics, elastic, lace material and the like that attaches, and adheres to couple with any existing garment 38 such as the design of a daily bra, brallete, sports bra, t-shirt bra, lingerie or similar top. This adjustable extension 400 becomes a component of a bra that is designed to match the bra pattern of any existing bra design so that the wings are the same length. Therefore, the adjustable extension 400 is coupled to an existing garment 38. A pair of complimentary adjustable connector/hooks 401 make the extension look like a single unit, essentially transforming any existing garment 38 or bra to a monitoring device by placing new hooks 401 to the existing bra that go over the bra extension as well. The adjustable bra extension 400 can have add-ons such as elastic and the connector/hooks 401 to make the bra extension tighter. When placed in the wearer 62 torso and when complimentary connector/hooks 401 are closed, electrically connected, hooked and the garment 38 is touching the wearer's body the wearable monitoring device is activated. When the connector/hooks 401 of the bra are open, electrically disconnected, unhooked and lose the device turns Power OFF. The adjustable extension 400 can be coupled to garment 38 solely from the connector/hooks 401. Having connector/hooks 401 in the extremities, the extension can go around the wearers torso or around other body parts one or multiple times to tighten, fasten the extension and connect to any garment 38 or bra with a connector/hooks 401. Comprising sensors 40, distributed along the locations for body measurements, once fastened with the adjustable connector/hooks 401 sensors 40 will be firmly touching against skin of the wearer 38. In an exemplary embodiment, a pad 410 can improve contact between the sensors 40 and skin and reduce movement of the sensors 40 on the skin. The pad 410 can comprise a stay-put washable and reusable silicone lining, a silicone band, silicone dots or any silicon pattern or Silica gel glue, and/or adhesive and the like placed around sensors 40. Combination of similar materials around the sensors 40 and throughout the adjustable extension 400 can be used as long as they will not cause irritation, redness, itching or other side effects. Using only non-allergenic materials to skin that allow friction so when the sensor that contacts the skin and is around the fabrics or lace top hugs the torso/chest or other part of the body of wearer 62 so that adjustable extension 400 stays put and noise to sensors 40 is reduced.

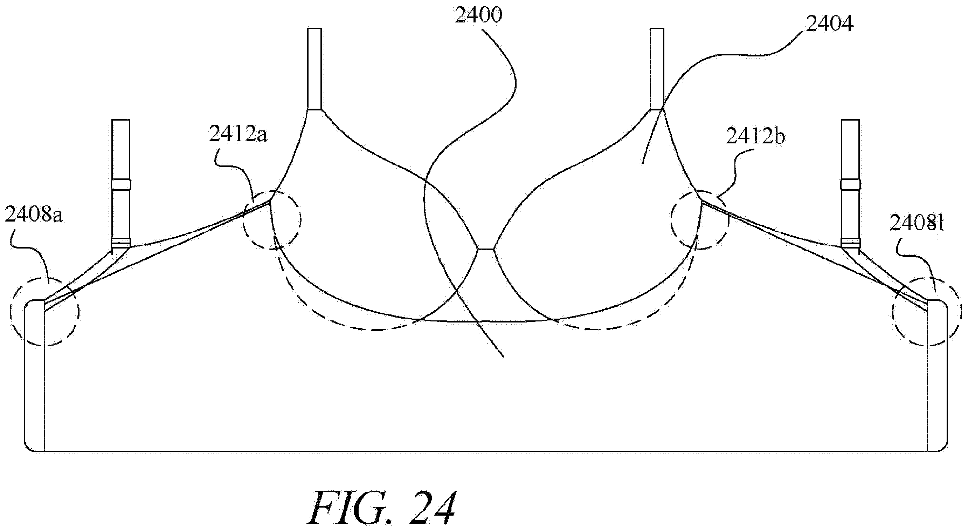

Referring now to FIG. 24, a schematic illustration of an exemplary garment extension 2400 as described above is illustrated. Garment extension 2400 may include, incorporate, and/or be attached to any element described above, including without limitation a printed circuit board 12 and/or any other element of a wearable monitoring device 10 as described above. Garment extension 2400 may be attached to a garment 2404. Garment 2404 may include, for example, a brassiere. Garment extension 2400 may be attached to garment 2404 at one or more distinct points, which may be referred to in this disclosure as "anchor points." Each anchor point may be separated from each other anchor point; in other words, there may not be a continuous seam connecting one anchor point to another. Separation between anchor points may allow for flexible attachment between garment 2404 and extension 2400, permitting attachment of extension 2400 to various kinds of garment and/or brassiere. This may enable extension 2400 to act as a universal extension of various garments of a given type, such as various brassieres and/or types of brassieres. Anchor points may include a first pair of anchor points 2408a-b located at extrema of a garment to clasp about a torso or other body part; for instance, first pair of anchor points 2408a-b may be at two sets of brassiere coupling hooks that clasp at the back of a person wearing the brassiere. Alternatively or additionally, for instance, where the garment clasps at the front of a person wearing the garment, first pair of anchor points 2408a-b may be located at a point at the center of the person's back, such as a point corresponding to a location where a rear-clasping bra would be clasped. Extension 2400 may include a second pair of anchor points 2412a-b. Second pair of anchor points 2412a-b may be located ventrally with respect to the torso of wearer; that is, the second pair 2412a-b may be located at points of garment 2404 that may touch skin over the chest wall, ventral ribs, breastbone, breasts, nipples, or the like. For instance, and without limitation, second pair 2412a-b may be located around edges of cups of a brassiere, such as outer edges of the cups, where inner edges are close to each other at the center of the chest of the user while outer edges are farther apart and toward the sides of the torso, or between the nipples and the armpits. In an embodiment, extension may be attached to a brassiere at four discrete points, the four discrete points including two points 2412a-b at edges of brassiere cups and two points 2408a-b at brassiere coupling hooks.

Still referring to FIG. 24, anchor points may be attached using any form of attachment as described above in reference to FIGS. 1-23. For instance anchor points may be attached using stitching or suturing, adhesion, hooks, snaps, rivets, slide fasteners such as zippers, and/or press fasteners. As used herein, a press fastener is a fastener that couples a first surface to a second surface when the two surfaces are pressed together. Some press fasteners include elements on the first surface that interlock with elements on the second surface; such fasteners include without limitation hook-and-loop fasteners such as VELCRO fasteners produced by Velcro Industries B.V. Limited Liability Company of Curacao Netherlands, and fasteners held together by a plurality of flanged or "mushroom"-shaped elements, such as 3M DUAL LOCK fasteners manufactured by 3M Company of Saint Paul, Minnesota. Press-fastener may also include adhesives, including reusable gel adhesives, GECKSKIN adhesives developed by the University of Massachusetts in Amherst, of Amherst, Massachusetts, or other reusable adhesives. Where press-fastener includes an adhesive, the adhesive may be entirely located on the first surface of the press-fastener or on the second surface of the press-fastener, allowing any surface that can adhere to the adhesive to serve as the corresponding surface. Any attachment between elements as described above may include and/or be performed with a press fastener.

It is to be noted that any one or more of the aspects and embodiments described herein may be conveniently implemented using one or more machines (e.g., one or more computing devices that are utilized as a user computing device for an electronic document, one or more server devices, such as a document server, etc.) programmed according to the teachings of the present specification, as will be apparent to those of ordinary skill in the computer art. Appropriate software coding can readily be prepared by skilled programmers based on the teachings of the present disclosure, as will be apparent to those of ordinary skill in the software art. Aspects and implementations discussed above employing software and/or software modules may also include appropriate hardware for assisting in the implementation of the machine executable instructions of the software and/or software module.

Such software may be a computer program product that employs a machine-readable storage medium. A machine-readable storage medium may be any medium that is capable of storing and/or encoding a sequence of instructions for execution by a machine (e.g., a computing device) and that causes the machine to perform any one of the methodologies and/or embodiments described herein. Examples of a machine-readable storage medium include, but are not limited to, a magnetic disk, an optical disc (*e.g.,* CD, CD-R, DVD, DVD-R, etc.), a magneto-optical disk, a read-only memory "ROM" device, a random access memory "RAM" device, a magnetic card, an optical card, a solid-state memory device, an EPROM, an EEPROM, and any combinations thereof. A machine-readable medium, as used herein, is intended to include a single medium as well as a collection of physically separate media, such as, for example, a collection of compact discs or one or more hard disk drives in combination with a computer memory. As used herein, a machine-readable storage medium does not include transitory forms of signal transmission.

Such software may also include information (*e.g.,* data) carried as a data signal on a data carrier, such as a carrier wave. For example, machine-executable information may be included as a data-carrying signal embodied in a data carrier in which the signal encodes a sequence of instruction, or portion thereof, for execution by a machine (*e.g.,* a computing device) and any related information (*e.g.,* data structures and data) that causes the machine to perform any one of the methodologies and/or embodiments described herein.

Examples of a computing device include, but are not limited to, an electronic book reading device, a computer workstation, a terminal computer, a server computer, a handheld device (*e.g.,* a tablet computer, a smartphone, etc.), a web appliance, a network router, a network switch, a network bridge, any machine capable of executing a sequence of instructions that specify an action to be taken by that machine, and any combinations thereof. In one example, a computing device may include and/or be included in a kiosk.

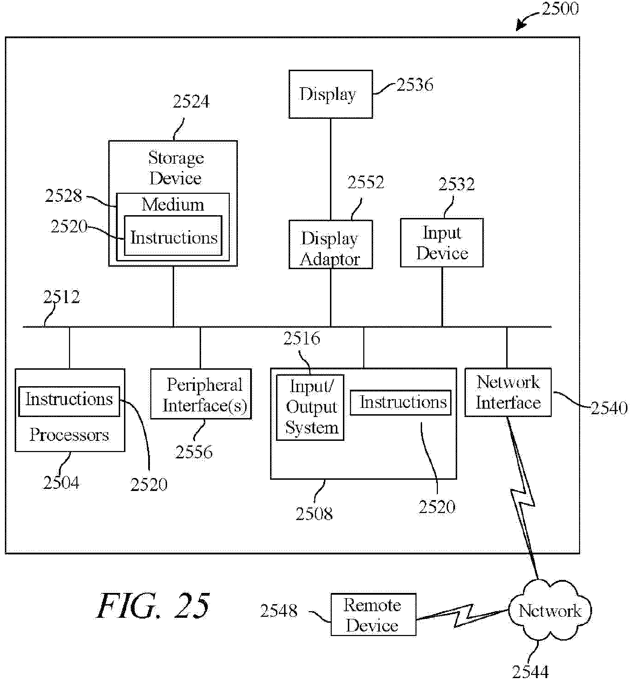

FIG. 25 shows a diagrammatic representation of one embodiment of a computing device in the exemplary form of a computer system 2500 within which a set of instructions for causing a control system to perform any one or more of the aspects and/or methodologies of the present disclosure may be executed. It is also contemplated that multiple computing devices may be utilized to implement a specially configured set of instructions for causing one or more of the devices to perform any one or more of the aspects and/or methodologies of the present disclosure. Computer system 2500 includes a processor 2504 and a memory 2508 that communicate with each other, and with other components, via a bus 2512. Bus 2512 may include any of several types of bus structures including, but not limited to, a memory bus, a memory controller, a peripheral bus, a local bus, and any combinations thereof, using any of a variety of bus architectures.

Processor 2504 may include any suitable processor, such as without limitation a processor incorporating logical circuitry for performing arithmetic and logical operations, such as an arithmetic and logic unit (ALU), which may be regulated with a state machine and directed by operational inputs from memory and/or sensors; processor 2504 may be organized according to Von Neumann and/or Harvard architecture as a non-limiting example. Processor 2504 may include, incorporate, and/or be incorporated in, without limitation, a microcontroller, microprocessor, digital signal processor (DSP), Field Programmable Gate Array (FPGA), Complex Programmable Logic Device (CPLD), Graphical Processing Unit (GPU), general purpose GPU, Tensor Processing Unit (TPU), analog or mixed signal processor, Trusted Platform Module (TPM), a floating point unit (FPU), and/or system on a chip (SoC)

Memory 2508 may include various components (*e.g.,* machine-readable media) including, but not limited to, a random-access memory component, a read only component, and any combinations thereof. In one example, a basic input/output system 2516 (BIOS), including basic routines that help to transfer information between elements within computer system 2500, such as during start-up, may be stored in memory 2508. Memory 2508 may also include (*e.g.,* stored on one or more machine-readable media) instructions (*e.g.,* software) 2520 embodying any one or more of the aspects and/or methodologies of the present disclosure. In another example, memory 2508 may further include any number of program modules including, but not limited to, an operating system, one or more application programs, other program modules, program data, and any combinations thereof.

Computer system 2500 may also include a storage device 2524. Examples of a storage device (*e.g.,* storage device 2524) include, but are not limited to, a hard disk drive, a magnetic disk drive, an optical disc drive in combination with an optical medium, a solid-state memory device, and any combinations thereof. Storage device 2524 may be connected to bus 2512 by an appropriate interface (not shown). Example interfaces include, but are not limited to, SCSI, advanced technology attachment (ATA), serial ATA, universal serial bus (USB), IEEE 1394 (FIREWIRE), and any combinations thereof. In one example, storage device 2524 (or one or more components thereof) may be removably interfaced with computer system 2500 (*e.g.,* via an external port connector (not shown)). Particularly, storage device 2524 and an associated machine-readable medium 2528 may provide nonvolatile and/or volatile storage of machine-readable instructions, data structures, program modules, and/or other data for computer system 2500. In one example, software 2520 may reside, completely or partially, within machine-readable medium 2528. In another example, software 2520 may reside, completely or partially, within processor 2504.

Computer system 2500 may also include an input device 2532. In one example, a user of computer system 2500 may enter commands and/or other information into computer system 2500 via input device 2532. Examples of an input device 2532 include, but are not limited to, an alphanumeric input device (*e.g.,* a keyboard), a pointing device, a joystick, a gamepad, an audio input device (*e.g.,* a microphone, a voice response system, etc.), a cursor control device (*e.g.,* a mouse), a touchpad, an optical scanner, a video capture device (*e.g.,* a still camera, a video camera), a touchscreen, and any combinations thereof. Input device 2532 may be interfaced to bus 2512 via any of a variety of interfaces (not shown) including, but not limited to, a serial interface, a parallel interface, a game port, a USB interface, a FIREWIRE interface, a direct interface to bus 2512, and any combinations thereof. Input device 2532 may include a touch screen interface that may be a part of or separate from display 2536, discussed further below. Input device 2532 may be utilized as a user selection device for selecting one or more graphical representations in a graphical interface as described above.

A user may also input commands and/or other information to computer system 2500 via storage device 2524 ( e.g ., a removable disk drive, a flash drive, etc.) and/or network interface device 2540. A network interface device, such as network interface device 2540, may be utilized for connecting computer system 2500 to one or more of a variety of networks, such as network 2544, and one or more remote devices 2548 connected thereto. Examples of a network interface device include, but are not limited to, a network interface card (e.g., a mobile network interface card, a LAN card), a modem, and any combination thereof. Examples of a network include, but are not limited to, a wide area network (e.g, the Internet, an enterprise network), a local area network (e.g, a network associated with an office, a building, a campus or other relatively small geographic space), a telephone network, a data network associated with a telephone/voice provider (e.g., a mobile communications provider data and/or voice network), a direct connection between two computing devices, and any combinations thereof. A network, such as network 2544, may employ a wired and/or a wireless mode of communication. In general, any network topology may be used. Information (e.g, data, software 2520, etc.) may be communicated to and/or from computer system 2500 via network interface device 2540.

Computer system 2500 may further include a video display adapter 2552 for communicating a displayable image to a display device, such as display device 2536. Examples of a display device include, but are not limited to, a liquid crystal display (LCD), a cathode ray tube (CRT), a plasma display, a light emitting diode (LED) display, and any combinations thereof. Display adapter 2552 and display device 2536 may be utilized in combination with processor 2504 to provide graphical representations of aspects of the present disclosure. In addition to a display device, computer system 2500 may include one or more other peripheral output devices including, but not limited to, an audio speaker, a printer, and any combinations thereof. Such peripheral output devices may be connected to bus 2512 via a peripheral interface 2556. Examples of a peripheral interface include, but are not limited to, a serial port, a USB connection, a FIREWIRE connection, a parallel connection, and any combinations thereof.

The foregoing has been a detailed description of illustrative embodiments of the invention. Various modifications and additions can be made. Features of each of the various embodiments described above may be combined with features of other described embodiments as appropriate in order to provide a multiplicity of feature combinations in associated new embodiments. Furthermore, while the foregoing describes a number of separate embodiments, what has been described herein is merely illustrative of the application of the principles of the present invention. Additionally, although particular methods herein may be illustrated and/or described as being performed in a specific order, the ordering is highly variable within ordinary skill to achieve embodiments according to the present disclosure..

Exemplary embodiments have been disclosed above and illustrated in the accompanying drawings. It will be understood by those skilled in the art that various changes, omissions and additions may be made to that which is specifically disclosed herein within the scope of the appended claims.

## Claims

1. A garment (38) having a wearable monitoring device, the garment comprising:
the wearable monitoring device (10) coupled to the garment, wherein:
the wearable monitoring device comprises a circuit board (12) having a first side and a second side opposite the first side;
the circuit board comprises an integrated circuit configured to process physiological measurement and wirelessly transmit the physiological measurements and biometric measurements to another computing device;
at least one soft-based module coupled to the wearable monitoring device and the garment, the at least one soft-based module configured to monitor a physiological condition through inputs of the physiological measurements, wherein the at least on soft-based module includes at least one sensor (40) configured to make the physiological measurements, **characterised in that**: the garment comprises complimentary hooks (401) configured to activate the wearable monitoring device responsive to the complimentary hooks making electrical contact.

2. The garment of claim 1, wherein the garment is configured to fit over a torso of a wearer.

3. The garment of claim 2, wherein the garment is a brassiere.

4. The garment of claim 1, wherein the at least one soft-based module has a biopotential sensor that enables biopotential measurements.

5. The garment of claim 1, wherein the circuit board is coupled to a conductive layer that contacts a wearer's skin, the garment further comprising a conductive pad coupled to the printed circuit board, wherein:
the conductive pad has at least dime-size dimensions; and
the conductive layer has dimensions greater than or equal to the dimensions of the conductive pad.

6. The garment of claim 5, wherein the conductive layer includes at least a first element on a portion of the garment configured to be worn on a ventral portion of a torso or at least a second element on a portion of the garment configured to be worn on a lateral portion of a torso.

7. The garment of claim 5, wherein the conductive layer that contacts the wearers skin comprises a conductive material including silverized thread or stretch conductive fabric or silver plated material or tin coated material or copper coated material or cobalt alloy top coating or silver plated knitted fabric or silver chloride-plated knitted fabric.

8. The garment of claim 1, wherein the garment has a shape of an adjustable bra extension.

9. The garment of claim 8, wherein the adjustable bra extension is attached to a brassiere at four discrete points, the four discrete points including two points at edges of brassiere cups and two points at brassiere coupling hooks.

10. The garment of claim 1, wherein the at least one soft-based module coupled to the wearable monitoring device and the garment comprises at least one of a long-term body monitoring system for at least one of: a health state and a disease.

11. The garment of claim 1, wherein the at least one soft-based module comprises:
a front layer coupled to a conductive tape, the front layer configured to directly contact skin of a wearer;
a circuit layer coupled to the conductive tape opposite the front layer; and
a padding layer coupled to the circuit layer opposite the conductive tape, wherein the padding layer is configured to attach to the garment.

12. The garment of claim 1, wherein the at least one soft-based module comprises:
an air channel layer coupled to the circuit layer proximate the front layer;
a fluid tray layer coupled to the air channel layer; and
a valve layer coupled to the fluid tray layer; wherein the air channel layer, fluid tray layer and valve layer are configured to make fluidic measurements.

13. The garment of claim 1, wherein the at least one soft-based module comprises at least one of: a piezo electric layer coupled between the front layer and the circuit layer, wherein the piezo electric layer is configured to make mechanical pressure measurements or at least one soft-based module comprises at least one acoustic layer coupled between the front layer and the circuit layer, wherein the at least one acoustic layer is configured for sound measurements.

14. The garment of claim 1, wherein the at least one soft-based module comprises an I/O interactive sensor configured for touch and optical measurements and indications.

15. The garment of claim 1, further comprising a pad coupled to the garment proximate the at least one sensor.

## Patentansprüche

1. Kleidungsstück (38) mit einer tragbaren Überwachungsvorrichtung, wobei das Kleidungsstück umfasst:
die tragbare Überwachungsvorrichtung (10), die mit dem Kleidungsstück gekoppelt ist, wobei:
die tragbare Überwachungsvorrichtung eine Platine (12) mit einer ersten Seite und einer zweiten Seite gegenüber der ersten Seite umfasst;
die Platine eine integrierte Schaltung umfasst, die zum Verarbeiten einer physiologischen Messung und drahtlosen Übertragen der physiologischen Messungen und von biometrischen Messungen zu einer weiteren Rechenvorrichtung ausgebildet ist;
mindestens ein Modul auf einer weichen Basis, das mit der tragbaren Überwachungsvorrichtung und dem Kleidungsstück gekoppelt ist, wobei das mindestens eine Modul auf einer weichen Basis zum Überwachen eines physiologischen Zustands durch Eingaben der physiologischen Messungen umfasst, wobei das mindestens eine Modul auf einer weichen Basis mindestens einen Sensor (40) umfasst, der zum Durchführen der physiologischen Messungen ausgebildet ist, **dadurch gekennzeichnet, dass**:
das Kleidungsstück komplementäre Haken (401) umfasst, die zum Aktivieren der tragbaren Überwachungsvorrichtung als Reaktion darauf, dass die komplementären Haken einen elektrischen Kontakt herstellen, ausgebildet sind.

2. Kleidungsstück nach Anspruch 1, wobei das Kleidungsstück zum Anlegen über einen Oberkörper eines Trägers ausgebildet ist.

3. Kleidungsstück nach Anspruch 2, wobei das Kleidungsstück ein Büstenhalter ist.

4. Kleidungsstück nach Anspruch 1, wobei das mindestens eine Modul auf einer weichen Basis einen Biopotenzialsensor aufweist, der Biopotenzialmessungen ermöglicht.

5. Kleidungsstück nach Anspruch 1, wobei die Platine mit einer leitenden Schicht gekoppelt ist, welche die Haut eines Trägers kontaktiert, wobei das Kleidungsstück ferner eine leitende Kontaktstelle umfasst, die mit der gedruckten Platine gekoppelt ist, wobei:
die leitende Kontaktstelle mindestens Abmessungen eines Zehncentstücks aufweist; und
die leitende Schicht Abmessungen aufweist, die größer als die Abmessungen der leitenden Kontaktstelle oder mit diesen identisch sind.

6. Kleidungsstück nach Anspruch 5, wobei die leitende Schicht mindestens ein erstes Element auf einem Abschnitt des Kleidungsstücks, der zum Tragen auf einem bauchseitigen Abschnitt eines Oberkörpers ausgebildet ist, oder mindestens ein zweites Element auf einem Abschnitt des Kleidungsstücks, der zum Tragen auf einem seitlichen Abschnitt eines Oberkörpers ausgebildet ist, umfasst.

7. Kleidungsstück nach Anspruch 5, wobei die leitende Schicht, welche die Haut des Trägers kontaktiert, ein leitendes Material umfasst, das einen mit Silber versehenen Faden oder ein leitendes Stretch-Gewebe oder ein versilbertes Material oder ein Zinnbeschichtetes Material oder ein Kupfer-beschichtetes Material oder eine Deckbeschichtung aus einer Kobaltlegierung oder ein versilbertes Gestrick oder ein Silberchlorid-plattiertes Gestrick umfasst.

8. Kleidungsstück nach Anspruch 1, wobei das Kleidungsstück die Form einer einstellbaren Büstenhalterverlängerung aufweist.

9. Kleidungsstück nach Anspruch 8, wobei die einstellbare Büstenhalterverlängerung an vier getrennten Punkten an einem Büstenhalter angebracht ist, wobei die vier getrennten Punkte zwei Punkte an Kanten von Büstenhaltercups und zwei Punkte an Büstenhalter-Kopplungshaken umfassen.

10. Kleidungsstück nach Anspruch 1, wobei das mindestens eine Modul auf einer weichen Basis mit der tragbaren Überwachungsvorrichtung gekoppelt ist und das Kleidungsstück mindestens eines von einem Langzeit-Körperüberwachungssystem für mindestens eines von: einem Gesundheitszustand und einer Erkrankung umfasst.

11. Kleidungsstück nach Anspruch 1, wobei das mindestens eine Modul auf einer weichen Basis umfasst:
eine vordere Schicht, die mit einem leitenden Band gekoppelt ist, wobei die vordere Schicht für ein direktes Kontaktieren der Haut einer Trägers ausgebildet ist;
eine Schaltungsschicht, die mit dem leitenden Band gegenüber der vorderen Schicht gekoppelt ist; und
eine Polsterungsschicht, die mit der Schaltungsschicht gegenüber dem leitenden Band gekoppelt ist, wobei die Polsterungsschicht zum Anbringen an das Kleidungsstück ausgebildet ist.

12. Kleidungsstück nach Anspruch 1, wobei das mindestens eine Modul auf einer weichen Basis umfasst:
eine Luftkanalschicht, die mit der Schaltungsschicht benachbart zu der vorderen Schicht gekoppelt ist;
eine Fluideinsatzschicht, die mit der Luftkanalschicht gekoppelt ist; und
eine Ventilschicht, die mit der Fluideinsatzschicht gekoppelt ist; wobei die Luftkanalschicht, die Fluideinsatzschicht und die Ventilschicht zum Durchführen von Strömungsmessungen ausgebildet sind.

13. Kleidungsstück nach Anspruch 1, wobei das mindestens eine Modul auf einer weichen Basis mindestens eines von: einer piezoelektrischen Schicht, die zwischen der vorderen Schicht und der Schaltungsschicht gekoppelt ist, wobei die piezoelektrische Schicht zur Durchführung von mechanischen Druckmessungen ausgebildet ist, oder mindestens einem Modul auf einer weichen Basis, das mindestens eine Akustikschicht umfasst, die zwischen der vorderen Schicht und der Schaltungsschicht gekoppelt ist, wobei die mindestens eine Akustikschicht für Schallmessungen ausgebildet ist, umfasst.

14. Kleidungsstück nach Anspruch 1, wobei das mindestens eine Modul auf einer weichen Basis einen interaktiven E/A-Sensor umfasst, der für Berührungs- und Optikmessungen und -angaben ausgebildet ist.

15. Kleidungsstück nach Anspruch 1, das ferner eine Kontaktstelle umfasst, die mit dem Kleidungsstück benachbart zu dem mindestens einen Sensor gekoppelt ist.

## Revendications

1. Vêtement (38) ayant un dispositif de surveillance portable, le vêtement comprenant :
le dispositif de surveillance portable (10) couplé au vêtement, dans lequel :
le dispositif de surveillance portable comprend une carte de circuit (12) ayant un premier côté et un second côté opposé au premier côté ;
la carte de circuit comprend un circuit intégré configuré pour traiter une mesure physiologique et transmettre sans fil les mesures physiologiques et des mesures biométriques à un autre dispositif informatique ;
au moins un module à base de logiciel couplé au dispositif de surveillance portable et au vêtement, l'au moins un module à base de logiciel étant configuré pour surveiller une condition physiologique par des entrées des mesures physiologiques, dans lequel l'au moins un module à base de logiciel inclut au moins un capteur (40) configuré pour réaliser les mesures physiologiques, **caractérisé en ce que** :
le vêtement comprend des crochets gratuits (401) configurés pour activer le dispositif de surveillance portable en réponse aux crochets gratuits faisant un contact électrique.

2. Vêtement selon la revendication 1, dans lequel le vêtement est configuré pour s'ajuster sur un torse d'un porteur.

3. Vêtement selon la revendication 2, dans lequel le vêtement est une brassière.

4. Vêtement selon la revendication 1, dans lequel l'au moins un module à base de logiciel a un capteur de biopotentiel qui permet des mesures de biopotentiel.

5. Vêtement selon la revendication 1, dans lequel la carte de circuit est couplée à une couche conductrice qui vient en contact avec la peau d'un porteur, le vêtement comprenant en outre un plot conducteur couplé à la carte de circuit imprimé, dans lequel :
le plot conducteur a au moins des dimensions de la taille d'une pièce de dix cents ; et
la couche conductrice a des dimensions supérieures ou égales aux dimensions du plot conducteur.

6. Vêtement selon la revendication 5, dans lequel la couche conductrice inclut au moins un premier élément sur une portion du vêtement configuré pour être porté sur une portion ventrale d'un torse ou au moins un second élément sur une portion du vêtement configuré pour être porté sur une portion latérale d'un torse.

7. Vêtement selon la revendication 5, dans lequel la couche conductrice qui vient en contact avec la peau du porteur comprend un matériau conducteur incluant un fil argenté ou tissu conducteur extensible ou matériau en argent plaqué ou matériau enduit d'étain ou matériau enduit de cuivre ou revêtement supérieur en alliage de cobalt ou tissu tricoté en argent plaqué ou tissu tricoté en chlorure d'argent plaqué.

8. Vêtement selon la revendication 1, dans lequel le vêtement a une forme d'une rallonge de soutien-gorge ajustable.

9. Vêtement selon la revendication 8, dans lequel la rallonge de soutien-gorge ajustable est reliée à une brassière en quatre points distincts, les quatre points distincts incluant deux points à des bords de bonnets de brassière et deux points au niveau de crochets d'attache de brassière.

10. Vêtement selon la revendication 1, dans lequel l'au moins un module à base de logiciel couplé au dispositif de surveillance portable et au vêtement comprend au moins un d'un système de surveillance corporelle à long terme pour au moins un élément parmi : un état de santé et une maladie.

11. Vêtement selon la revendication 1, dans lequel l'au moins un module à base de logiciel comprend :
une couche avant couplée à un ruban conducteur, la couche avant étant configurée pour venir directement en contact avec la peau d'un porteur ;
une couche de circuit couplée au ruban conducteur à l'opposé de la couche avant ; et
une couche de rembourrage couplée à la couche de circuit à l'opposé du ruban conducteur, dans lequel la couche de rembourrage est configurée pour s'attacher au vêtement.

12. Vêtement selon la revendication 1, dans lequel le module à base de logiciel comprend :
une couche de canal d'air couplée à la couche de circuit à proximité de la couche avant ;
une couche de plateau fluidique couplée à la couche de canal d'air ; et
une couche de vanne couplée à la couche de plateau fluidique ; dans lequel les couche de canal d'air, couche de plateau fluidique et couche de vanne sont configurées pour réaliser des mesures fluidiques.

13. Vêtement selon la revendication 1, dans lequel l'au moins un module à base de logiciel comprend au moins une de : une couche piézoélectrique couplée entre la couche avant et la couche de circuit,
dans lequel la couche piézoélectrique est configurée pour réaliser des mesures de pression mécanique ou au moins un module à base de logiciel comprend au moins une couche acoustique couplée entre la couche avant et la couche de circuit, dans lequel l'au moins une couche acoustique est configurée pour des mesures sonores.

14. Vêtement selon la revendication 1, dans lequel l'au moins un module à base de logiciel comprend un capteur interactif E/S configuré pour des mesures et indications tactiles et optiques.

15. Vêtement selon la revendication 1, comprenant en outre un plot couplé au vêtement à proximité de l'au moins un capteur.
